# EUROPEAN PATENT APPLICATION

(11) **EP 0 658 567 A1**
(43) Date of publication of application: **21.06.1995**
(21) Application number: 94309168.6
(22) Date of filing: 08.12.1994
(51) Int. Cl.: C07K 14/42, C07K 14/47, C07K 14/77, C07K 16/44

(54) **Complexes between lectins and glycoproteins**

(30) Priority: 08.12.1993 JP 308418/93
(71) Applicant: RESEARCH DEVELOPMENT CORPORATION OF JAPAN, Kawaguchi-shi, Saitama-ken 332 (JP)
(72) Inventor: Matsubara,Keiko, Inashiki-gun Ibaraki (JP); Yamaki,Mariko, Tsukuba-shi Ibaraki (JP); Ebina,Satoshi, Tsukuba-shi Ibaraki (JP)
(74) Representative: Matthews, Derek Peter

(57) **Abstract**

The present invention provides an artificial protein supramolecule in which a protein molecule is bonded via a sugar chain to the surface of another protein molecule, and a method for forming thereof which comprises the steps of mixing protein molecules and causing bonding between them by the sugar molecular chain.

## Description

### FIELD OF THE INVENTION

The present invention relates to an artificial protein supramolecule. More particularly, the present invention relates to an artificial protein supramolecule useful as a novel functional artificial molecular structure in such various areas as medicine, biologically compatible materials, bioelectronics and chemical industry.

### PRIOR ART AND PROBLEMS

Protein molecules are attracting general attention as permitting formation not only of medical and biologically compatible materials but also artificial material structures having such advanced functions as bioelements, material segregation and material recognition. Searching therefor, structural control and application thereof are causing growth of a unique area of research and development known as protein engineering.

This general attention given to protein molecules is based on the fact that the order and functions of material structures which seem to be mysterious in nature largely depend upon protein. Actually, there are innumerable kinds of protein complexes in nature. Protein itself displays sophisticated functions, and furthermore provides more complicated functions by forming complexes with protein of the same or a different kind. Hemoglobin, for example, provides bonding and dissociation reactions of oxygen suitable in vivo by combining four protein molecules having a molecular weight of 18,000. Ferritin stores several thousand iron atoms by combining 24 molecules having a molecule weight of 18,000. It is also known that many membrane proteins taking charge of complicated reactions intrinsic to a biological body form complexes (Molecular Biology of The Cell (2nd edition), Garland Publishing, Inc., NY.).

A complex thus comprising protein in a structure having a high function, may be referred to as a protein supramolecule.

Research on the functions and structures of these innumerable protein supramolecules present in nature is a new area of research. If it were possible to freely manufacture such protein complexes, on the other hand, there would be innumerable potential applications. There has however as yet been no full-scale trial to do so, and no case of success has as yet been reported.

### SUMMARY OF THE INVENTION

Under such circumstances, the present invention has an object to overcome restrictions in the prior art, artificially form an arbitrary protein molecular complex, and provide same as a novel artificial protein supramolecule. Another object of the present invention is to provide a method for this purpose.

Namely, as means to solve the above-mentioned problems, the present invention provides an artificial protein supramolecule in which protein molecules are bonded via a sugar chain on the surface of one of the protein molecules.

The present invention provides also a method for manufacturing an artificial protein supramolecule which comprises the steps of mixing a protein molecule having a sugar chain on the surface thereof with a sugar recognizing protein molecule specifically bonding to said sugar chain, and causing bonding thereof.

### BRIEF DESCRIPTION OF THE DRAWINGS

Figure 1 shows a configuration diagram illustrating a supramolecule of concanavalin A and ovalbumin;
Figure 2 shows an elution pattern diagram of gel filtration of a supramolecule of concanavalin A and ovalbumin;
Figure 3 shows a transmission electron microphotograph of the elution/fractionation shown in Fig. 2;
Figure 4 shows an elution pattern diagram of gel filtration resulting from dissociation of concanavalin A and ovalbumin;
Figure 5 shows a configuration diagram illustrating a supramolecule of WGA and ovalbumin;
Figure 6 shows a chemical structure diagram of an artificial albumin-complex;
Figure 7 shows a gel filtration/elution pattern diagram of a supramolecule of WGA and artificial egg white albumin;
Figure 8 shows a transmission electron microphotograph of the elution/fractionation shown in Fig. 7, and
Figure 9 shows an elution pattern diagram of gel filtration resulting from dissociation of an artificial aggregation into WGA and ovalbumin.

### DETAILED DESCRIPTION OF THE INVENTION

Many of the protein supramolecules present in nature are maintained by a force acting purely between protein molecules. Interaction between protein molecules involves such factors as attraction-type and repulsion-type interactions and biological compatibility, and it is difficult to artificially control these forces even with the present level of science and technology.

In the present invention, therefore, protein molecules are mutually bonded at a specific portion by a simple force similar to the relationship between a key and a keyhole.

An artificial protein supramolecule of the present invention, in general, comprises at least a protein having a sugar molecular chain on the surface thereof and at least a sugar molecular chain recognizing protein molecule. Said protein having a sugar molecular chain is bonded to said sugar molecular chain recognizing protein by said sugar molecular chain.

An artificial protein supramolecule of the present invention means a protein which is artificially formed and does not exist as a natural substance.

More particularly, bonding of protein molecules into a protein supramolecule occurs, not by interaction between protein molecules, but specifically via a sugar chain present on the surface of the protein molecule.

The principle is as follows. Some kinds of protein are known to manifest their functions through a specific bond to a sugar molecule (sugar recognizing protein). It is for example known that certain plant-originated low molecular weight proteins have a function of causing aggregation of blood cells (hemocyte aggregation). A protein having such a function is generally referred to as a lectin. Some kinds of lectin (Phaseolus vulgaris, PHA) are known to be bonded with a sugar molecule on the cell surface during interkinesis and accelerate division reactions (blastogenesis). At present, a similar mechanism in an animal cell which adjusts cell division is studied by some researchers (Molecular Biology of The Cell (2nd edition), Garland Publishing, Inc., N.Y.; Goldstein, I.J., Hughes, R.C., Monsigny, M., Osawa, T., Sharon, N. Nature, 258, 66 (1980)). There is anticipated even the existence of a protein which specifically recognizes a sugar molecule bonded to the receptor surface of a biomembrane, and controls a signal transmitting reaction. Many proteins in nature are known, on the other hand, to have a sugar molecule added after synthesis in vivo. Particularly, all blood proteins are believed to be glycoproteins.

In the present invention, a protein supramolecule is prepared by combining these sugar recognizing proteins with a sugar bonding protein.

In this case, an antibody which recognises a sugar chain as an antigen, may be utilized as lectin. Also some macroproteins create a protein supramolecule via a protein recognizing a sugar chain, called a linker, such a linker can be used as a lectin.

It is also possible to use a glycoprotein other than ovalbumin.

The sugar chain can be artificially bonded by the utilization of a specific reaction group on the protein surface, and a protein artificially bonded with a sugar chain, not a glycoprotein present in nature, can be used as a sugar bonding protein.

The present invention is described below further in detail by means of examples.

### Example 1

Rape-seed agglutinin (concanavalin A) was used as the sugar recognizing protein, and ovalbumin as the sugar bonding protein.

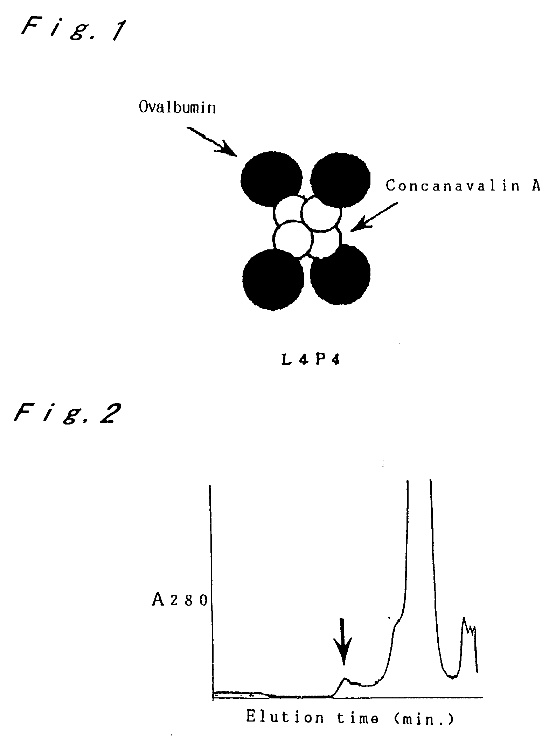

Concanavalin A is present in the form of a dimer having a molecular weight of 52,000 at a pH of up to 5.6, and in the form of a tetramer having a molecular weight of 104,000 at a pH of over 5.6. Each monomer of concanavalin A has a portion specifically bonded to mannose. At a pH near neutrality, therefore, an artificial supramolecule (L4P4) having four mannose-type proteins is expected, as shown in Fig. 1.

In Fig. 1, L represents lectin and P, protein.

Natural ovalbumin is a glycoprotein having a molecular weight of 45,000, and has a single sugar chain of the mannose type per molecule. Combination with concanavalin A is therefore preferable.

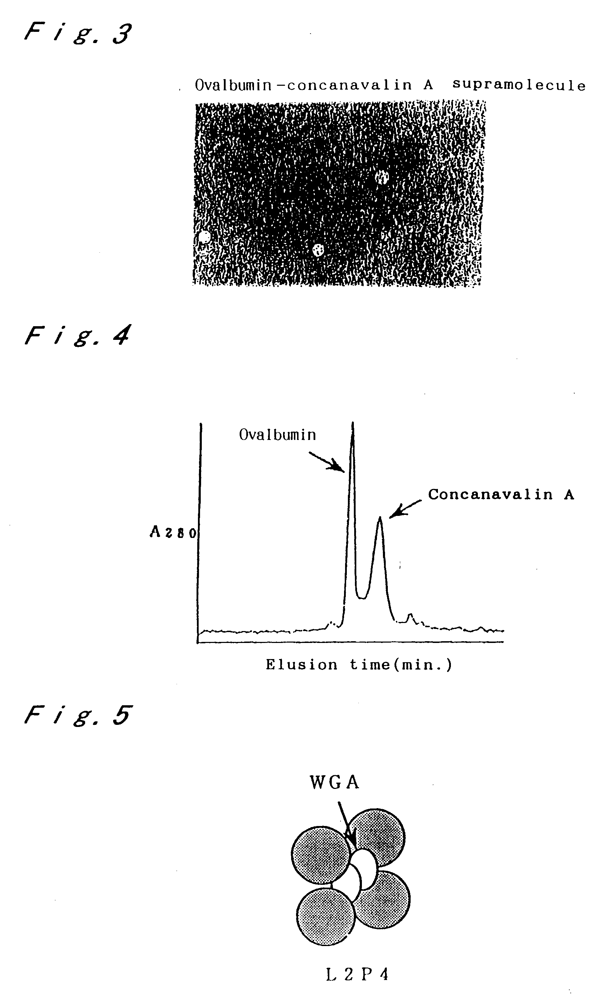

Concanavalin A and ovalbumin were mixed and subjected to gel filtration chromatography: a new peak was observed at a position marked with an arrow, as shown in Fig. 2. The state of elution and fractionation of this peak was observed with a transmission electron microscope. The result is shown in Fig. 3.

There was generated an artificial protein supramolecule of ovalbumin-concanavalin A. This was confirmed also by the fact that dissociation of ovalbumin from concanavalin A was observed, as shown in Fig. 4, by subjecting this supramolecule to a treatment with excess mannose.

### Example 2

Wheat germ agglutinin (WGA) was used as the sugar recognizing protein. This WGA is present in the form of a monomer having a molecular weight of 18,000 at an acidic pH, and in the form of a dimer within the neutral pH region, having two N-acetylglucosamine-type bonding portions per monomer. An artificial supramolecule (L2P4) having four N-acetylglucosamine-type glycoproteins bonded thereto is therefore expected at a pH near neutrality, where L represents lectin, and P, glycoprotein.

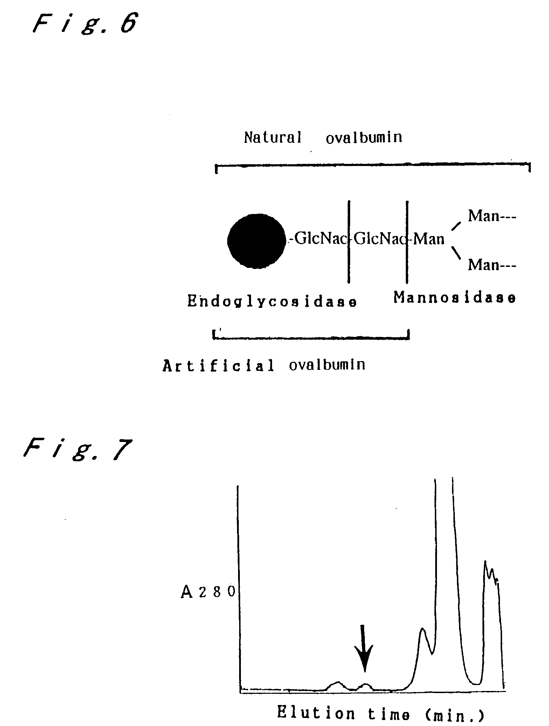

By causing an appropriate glycosidase (sugar chain digesting enzyme) to act, it is possible to prepare an N-acetylglucosamine-type ovalbumin (artificial ovalbumin), as shown in Fig. 6. In view of the satisfactory combination with WGA this artificial ovalbumin was mixed with WGA.

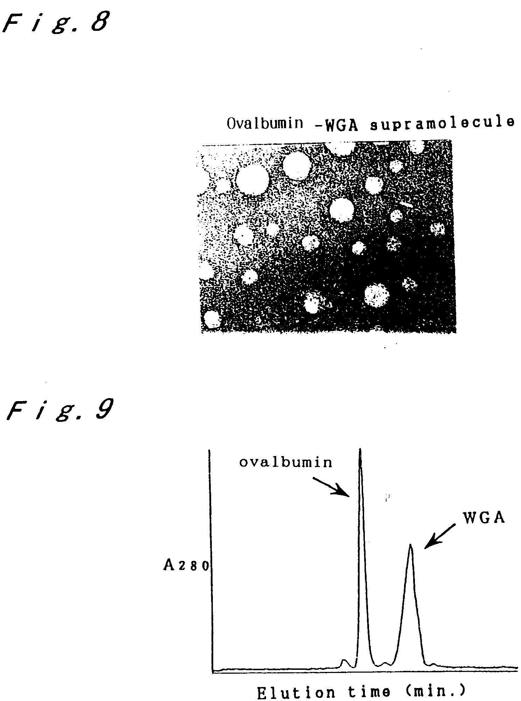

The resultant mixture was subjected to gel filtration chromatography: a new peak was observed at the position indicated by an arrow, as shown in Fig. 7. This fractionation was observed with a transmission electron microscope, as shown in Fig. 8.

An ovalbumin-WGA protein supramolecule was prepared. This was confirmed also by the fact that treatment of a fraction of this supramolecule with excess N-acetylglucosamine resulted in dissociation into ovalbumin and WGA, and an elution pattern of gel filtration was obtained.

As described above in detail, a novel protein supramolecule is provided by the present invention.

## Claims

1. An artificial protein supramolecule, which comprises one or more protein molecules, to which another protein molecule is artificially bonded via a sugar molecular chain on the surface of the protein molecule.

2. The artificial protein supramolecule claimed in claim 1, comprising a sugar molecular chain recognizing protein having an ability to bond selectively to a sugar molecule and a protein molecule having a sugar molecular chain on the surface thereof.

3. The artificial protein supramolecule claimed in claim 2, wherein the sugar molecular chain recognizing protein is a low molecular weight protein having a function of causing aggregation of blood cells.

4. The artificial protein supramolecule claimed in claim 3, wherein the low molecular weight protein having a function of causing aggregation of blood cells is a lectin.

5. The artificial protein supramolecule claimed in claim 2, wherein the sugar molecular chain recognizing protein is a protein derived from an animal cell.

6. The artificial protein supramolecule claimed in claim 2, wherein the sugar molecular chain recognizing protein is an antibody which recognizes a sugar molecular chain as an antigen, or is a linker.

7. The artificial protein supramolecule claimed in claim 2, wherein the protein molecule having a sugar molecular chain on the surface thereof is a blood protein.

8. A method for forming an artificial protein supramolecule according to any of claims 1 to 7, which comprises the steps of mixing protein molecules, and forming an artificial sugar molecular chain bond between said protein molecules.
